# EUROPEAN PATENT APPLICATION

(11) **EP 2 862 877 A1**
(43) Date of publication of application: **22.04.2015**
(21) Application number: 13306436.0
(22) Date of filing: 18.10.2013
(51) Int. Cl.: C07K 16/40, A61K 39/00

(54) **Methods for inhibiting atherosclerosis by administering an inhibitor of PCSK9**

(71) Applicant: SANOFI, 75008 Paris (FR)
(72) Inventor: The designation of the inventor has not yet been filed
(74) Representative: Then, Johann

(57) **Abstract**

The present invention provides methods and compositions for inhibiting atherosclerotic plaque formation in a subject. In certain embodiments, the methods of the present invention comprise selecting a subject who has, or is at risk of developing, atherosclerosis, and administering to the subject a pharmaceutical composition comprising a proprotein convertase subtilisin/kexin type 9 (PCSK9) inhibitor. In certain embodiments, the PCSK9 inhibitor is an anti-PCSK9 antibody, or antigen binding protein.

## Description

### RELATED APPLICATIONS

This application is related to USSN 61/832,459, filed June 7, 2013, the contents of which are hereby incorporated by reference in their entirety.

### FIELD OF THE INVENTION

The present invention relates to the field of therapeutic treatments for atherosclerosis. More specifically, the invention relates to the administration of proprotein convertase subtilisin/kexin type 9 (PCSK9) inhibitors to inhibit atherosclerotic plaque formation in a subject.

### BACKGROUND

Atherosclerosis represents the major cause of death and cardiovascular morbidity in the western world. Risk factors for atherosclerosis include high low density lipoprotein (LDL) cholesterol levels, low high density lipoprotein (HDL) cholesterol levels, hypertension, diabetes mellitus, family history, male gender, cigarette smoke, and high serum cholesterol.

Proprotein convertase subtilisin/kexin type 9 (PCSK9) is a proprotein convertase belonging to the proteinase K subfamily of the secretory subtilase family. It is a serine protease involved in LDL metabolism that is mainly expressed in the liver, kidney, and intestines. Evidence suggests that PCSK9 increases plasma LDL cholesterol by promoting degradation of the LDL receptor, which mediates LDL endocytosis in the liver, the major route of LDL clearance from circulation.

The use of PCSK9 inhibitors (anti-PCSK9 antibodies) to reduce serum total cholesterol, LDL cholesterol and serum triglycerides has been described in U.S. Patent Nos. 8,062,640, 8,357,371, and US Patent Application Publication No. 2013/0064834. Nonetheless, PCSK9 inhibitors have not been reported to reduce or inhibit progression of atherosclerotic plaque formation in a subject. There remains a need in the art for therapeutic methods of inhibiting atherosclerotic plaque formation.

### BRIEF SUMMARY OF THE INVENTION

The present invention addresses the need in the art for therapeutic methods by providing methods and compositions for inhibiting atherosclerotic plaque formation in a subject. In certain aspects, the methods of the present invention generally comprise selecting a subject who has, or is at risk of developing, atherosclerosis, and administering to the subject a pharmaceutical composition comprising a proprotein convertase subtilisin/kexin type 9 (PCSK9) inhibitor (e.g., an anti-PCSK9 antibody or antigen binding protein). In other aspects, the invention provides a pharmaceutical composition comprising a PCSK9 inhibitor for use in the inhibition of atherosclerotic plaque formation in a subject. In yet other aspects, the invention provides a method of inhibiting atherosclerotic plaque formation in a subject by administering to the subject a pharmaceutical composition comprising a PCSK9 inhibitor.

In certain embodiments, the subject is nonhyperlipidemic. In other embodiments, the subject is apparently healthy.

In another aspect, the invention provides a method of treating or inhibiting progression of atherosclerosis in a subject, the method comprising: (a) selecting a subject who has suffered a stroke or myocardial infarction; and (b) administering to the subject a pharmaceutical composition comprising a PCSK9 inhibitor, thereby treating or inhibiting progression of atherosclerosis. In one embodiment, the subject is nonhyperlipidemic.

In another aspect, the invention provides a method of treating or inhibiting progression of atherosclerosis in a nonhyperlipidemic subject, the method comprising (a) selecting a subject who has, or is known to be at risk of developing, atherosclerosis, wherein the subject is nonhyperlipidemic; and (b) administering to the subject a pharmaceutical composition comprising a PCSK9 inhibitor, thereby treating or inhibiting progression of atherosclerosis. In one embodiment, the subject is apparently healthy. In another embodiment, the subject is nonhypercholesterolemic. In another embodiment, the subject is nonhypertriglyceridemic.

In certain embodiments, the subject has a disease or disorder selected from the group consisting of type I diabetes mellitus, type II diabetes mellitus, Kawasaki disease, chronic inflammatory disease, and hypertension. In another embodiment, the subject has elevated levels of an inflammatory marker. In a further embodiment, the inflammatory marker is C-reactive protein. In another further embodiment, the inflammatory marker is an inflammatory cytokine.

In certain exemplary embodiments, the PCSK9 inhibitor is an antibody, or antigen binding protein, that specifically binds to PCSK9. In certain embodiments, the antibody comprises heavy and light chain CDR amino acid sequences having SEQ ID NOs:220, 222, 224, 228, 230 and 232. In certain embodiments, the antibody or antigen-binding protein comprises an HCVR having the amino acid sequence of SEQ ID NO:218 and an LCVR having the amino acid sequence of SEQ ID NO:226. In certain embodiments, the antibody or antigen-binding protein comprises heavy and light chain CDR amino acid sequences having SEQ ID NOs:76, 78, 80, 84, 86 and 88. In certain embodiments, the antibody or antigen-binding protein comprises an HCVR having the amino acid sequence of SEQ ID NO:90 and an LCVR having the amino acid sequence of SEQ ID NO:92. In one embodiment, the antibody or antigen-binding protein binds to the same epitope on PCSK9 as an antibody comprising the heavy and light variable domain amino acid sequences forth in SEQ ID NOs: 90 and 92; or 218 and 216, respectively. In some embodiments, the antibody or antigen-binding protein competes for binding to PCSK9 with an antibody comprising the heavy and light chain variable domain amino acid sequences forth in SEQ ID NOs: 90 and 92; or 218 and 216, respectively.

In another embodiment, the PCSK9 inhibitor reduces atherosclerotic plaque formation in the subject by at least 10%, 20%, 30%, 40%, 50%, 60%, 70%, 80%, or 90%.

In some embodiments, the subject has heterozygous Familial Hypercholesterolemia (heFH). In other embodiments, the subject has a form of hypercholesterolemia that is not Familial Hypercholesterolemia (nonFH).

In certain embodiments, the subject is on another lipid-modifying agent before and/or during administration of the antibody or antigen-binding protein. Therapeutic lipid-modifying agents include statins, cholesterol uptake inhibitors, ezetimibe, fibrates, niacin, omega-3 fatty acids, and bile acid resins. Statins include cerivastatin, atorvastatin, simvastatin, pitavastatin, rosuvastatin, fluvastatin, lovastatin and pravastatin.

In some embodiments, the antibody or antigen binding protein is administered subcutaneously.

The invention also provides a unit dosage form comprising the pharmaceutical compositions of PCSK9 inhibitors. In certain embodiments, the PCSK9 inhibitor is an antibody or antigen-binding fragment and the unit dosage form comprises 75 mg, 150 mg, 200 mg, or 300 mg of the antibody or antigen-binding fragment. The unit dosage form may be a pre-filled syringe, a pre-filled autoinjector, a vial, a cartridge, a reusable syringe, or a reusable autoinjector; the unit dosage form may be hermetically sealed, and may further indicate the dosage.

The invention also provides an article of manufacture or kit comprising the pharmaceutical composition of a PCSK9 inhibitor and a container, and in some embodiments also includes instructions for use.

### BRIEF DESCRIPTION OF THE DRAWINGS

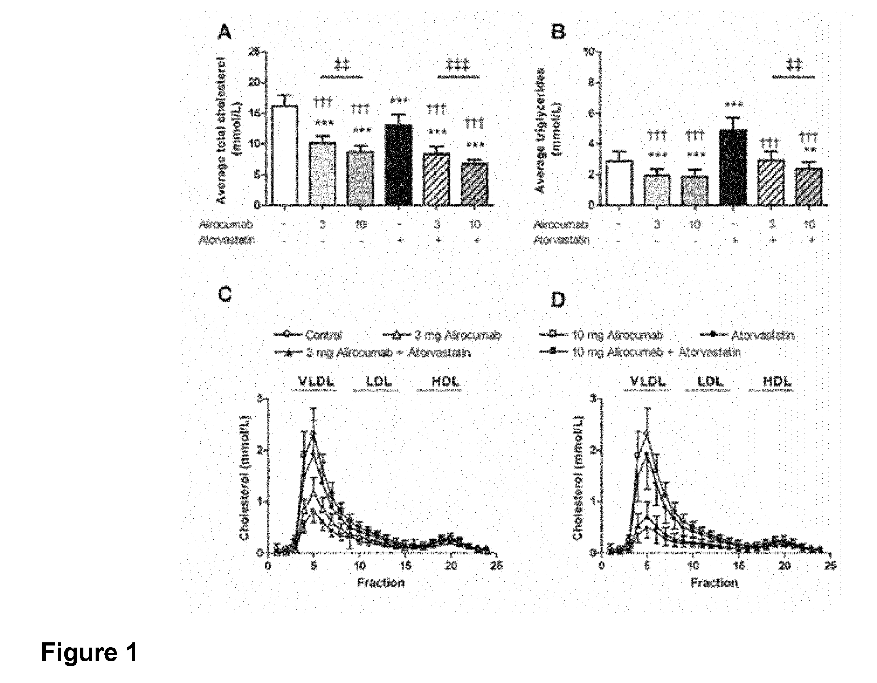

Figure 1 shows a series of graphs depicting the effect of alirocumab, atorvastatin, and their combination on several parameters after an 18 week treatment period. Figure 1A shows the effect on average plasma total cholesterol; Figure 1 B shows the effect on triglyceride levels. Figures 1C and 1D show the lipoprotein profiles for cholesterol as assessed by FPLC lipoprotein separation after 12 weeks of treatment to study the effects of alirocumab alone (1 C) and in combination with atorvastatin (1D). **P<0.01; ***P<0.001 as compared to control; †††P<0.001 as compared to atorvastatin; ‡‡P<0.01; ‡‡‡P<0.001 compared 3 mg/kg alirocumab to 10 mg/kg alirocumab (n=15 per group).

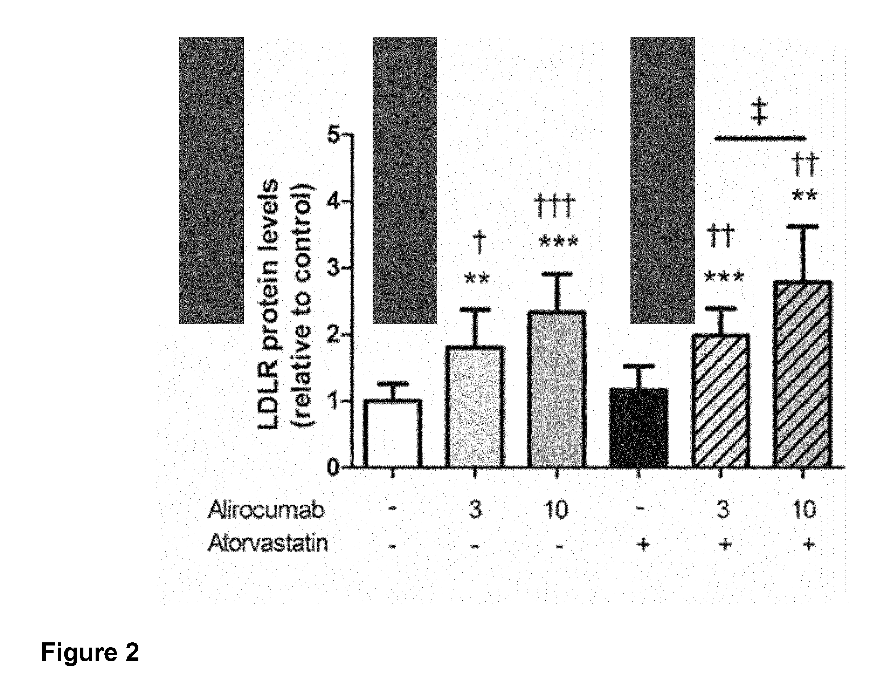

Figure 2 shows a graph of the effect of alirocumab, atorvastatin, and their combination on hepatic low density lipoprotein receptor protein levels. **P<0.01; ***P<0.001 as compared to control; †P<0.05; ††P<0.01; tttP<0.001 as compared to atorvastatin; ‡P<0.05 compared 3 mg/kg alirocumab to 10 mg/kg alirocumab (n=8 per group).

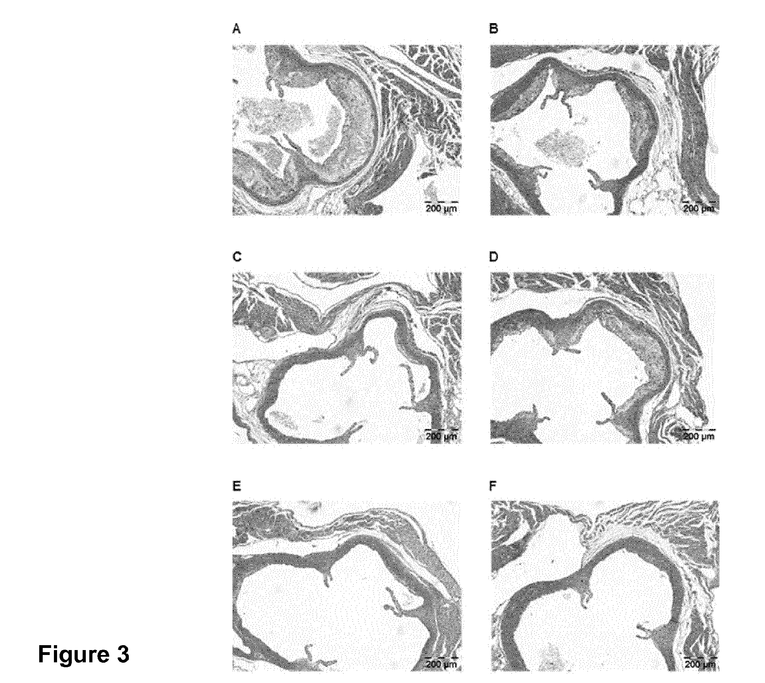

Figure 3 is a series of photos depicting the effect of alirocumab, atorvastatin, and their combination on plaque morphology. Representative images of hematoxylin-phloxine-saffron-stained atherosclerotic lesions in a cross section of the aortic root area for the control (A), 3 mg/kg alirocumab (B), 10 mg/kg alirocumab (C), atorvastatin (D), 3 mg/kg alirocumab + atorvastatin (E) and 10 mg/kg alirocumab + atorvastatin (F) groups, respectively, after 18 weeks of treatment.

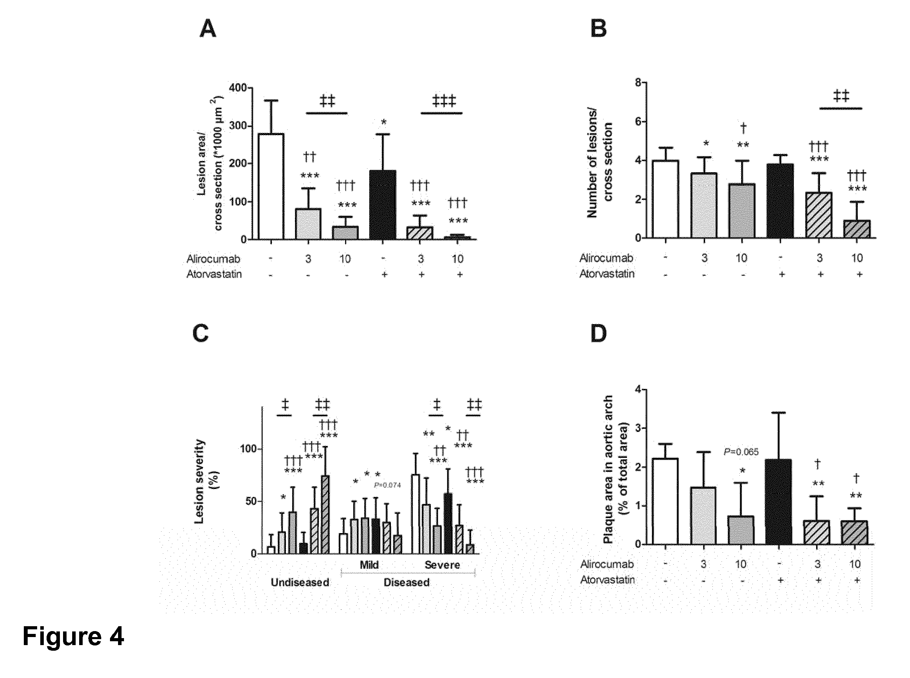

Figure 4 shows the effect of alirocumab, atorvastatin, and their combination on atherosclerosis development in aortic root and arch. After 18 weeks of treatment, the total lesion area (A) and number of lesions (B) per cross section were assessed. Lesion severity was assessed and categorized as no lesions, mild (type I-III) and severe (type IV-V) lesions (C). The total plaque load in the aortic arch (D) was analyzed after oil-red O-staining. Data are expressed as percentage of the stained area. *P<0.05; **P<0.01; ***P<0.001 as compared to control; †P<0.05; ††P<0.01; †††P<0.001 as compared to atorvastatin; ‡P<0.05; ‡‡P<0.01; ‡‡‡P<0.001 compared 3 mg/kg alirocumab to 10 mg/kg alirocumab (n=15 per group in the root area and n=6-7 in the arch).

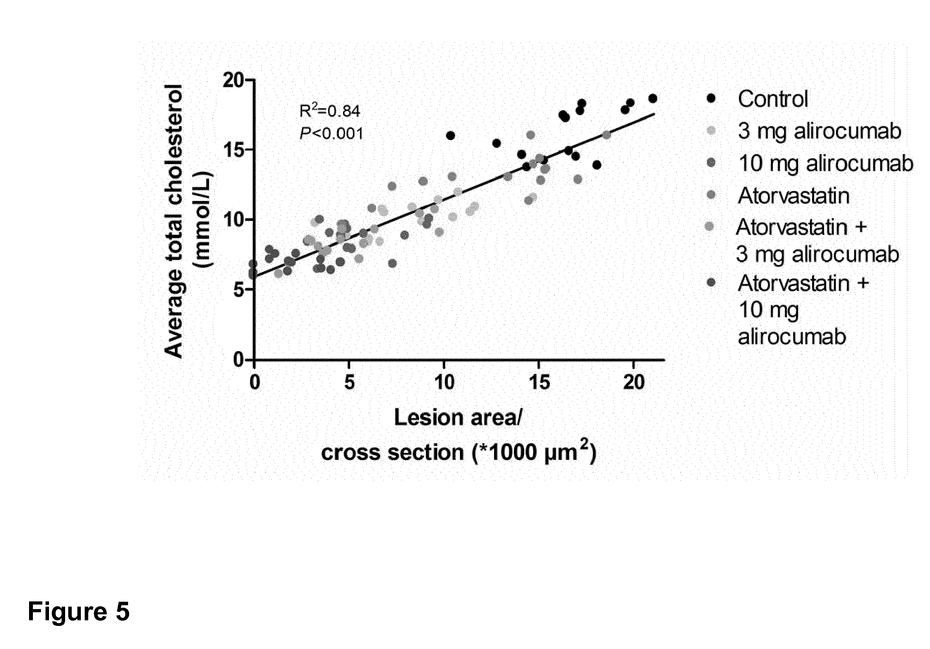

Figure 5 is a graph depicting the correlation between average plasma total cholesterol and atherosclerotic lesion area. The square root of the lesion area was plotted against average total cholesterol. Linear regression analysis was performed.

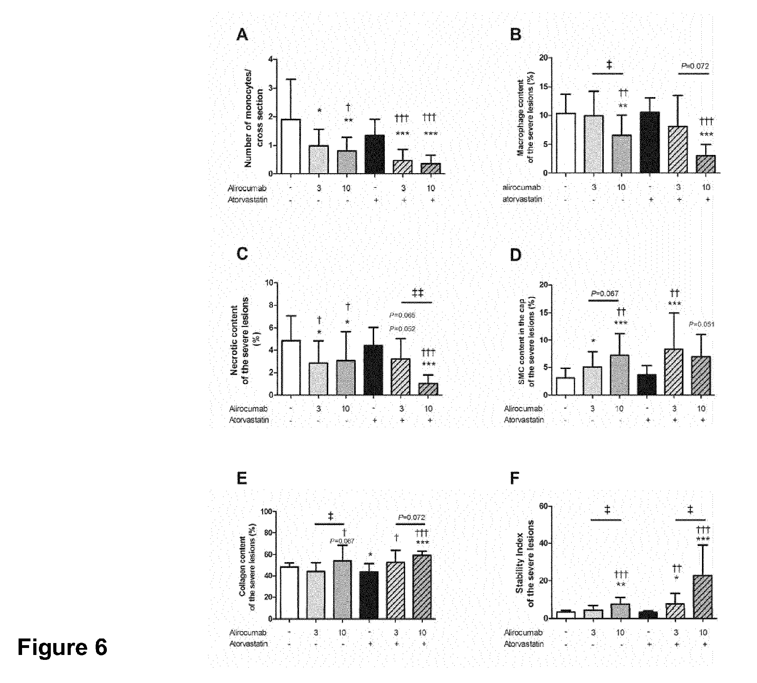

Figure 6 shows the effect of alirocumab, atorvastatin, and their combination on lesion composition. The number of monocytes adhering to the endothelium was determined per cross section (A). Macrophage (B) and necrotic content, including cholesterol clefts (C) as destabilization factors, and SMC (D) and collagen content (E) as stabilization factors were determined in the severe (type IV-V) lesions after correcting for lesion size. The plaque stability index was calculated as the ratio of the stabilization factors to the destabilization factors (F). *P<0.05; **P<0.01; ***P<0.001 as compared to control; †P<0.05; ††P<0.01; †††P<0.001; ‡P<0.05; ‡‡P<0.01 compared 3 mg/kg alirocumab to 10 mg/kg alirocumab (n=15 per group).

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice of the present invention, exemplary methods and materials are now described. All publications mentioned herein, and the sequence listing filed concurrently herewith, are incorporated herein by reference in their entirety.

### Methods for Inhibiting Atherosclerosis

The methods of the present invention comprise selecting subjects that have, or are at risk of developing, atherosclerosis, and administering to these subjects a pharmaceutical composition comprising a PCSK9 inhibitor.

Risk factors for atherosclerosis are well known in the art and include, without limitation, high low density lipoprotein (LDL) cholesterol levels, low high density lipoprotein (HDL) cholesterol levels, hypertension, diabetes mellitus, family history, male gender, cigarette smoking, and high serum cholesterol. Methods of assessing these risk factors for a given subject are also well known in the art.

In certain embodiments, the selected subject is nonhyperlipidemic. A "nonhyperlipidemic" is a subject that is a nonhypercholesterolemic and/or a nonhypertriglyceridemic subject. A "nonhypercholesterolemic" subject is one that does not fit the current criteria established for a hypercholesterolemic subject. A "nonhypertriglyceridemic" subject is one that does not fit the current criteria established for a hypertriglyceridemic subject (See, e.g., Harrison's Principles of Experimental Medicine, 13th Edition, McGraw-Hill, Inc., N.Y). A hypercholesterolemic subject has an LDL level of >160 mg/dL, or >130 mg/dL and at least two risk factors selected from the group consisting of male gender, family history of premature coronary heart disease, cigarette smoking (more than 10 per day), hypertension, low HDL (<35 mg/dL), diabetes mellitus, hyperinsulinemia, abdominal obesity, high lipoprotein (a), and personal history of cerebrovascular disease or occlusive peripheral vascular disease. A hypertriglyceridemic subject has a triglyceride (TG) level of >250 mg/dL. Thus, a nonhyperlipidemic subject is defined as one whose cholesterol and triglyceride levels are below the limits set as described above for both the hypercholesterolemic and hypertriglyceridemic subjects. In certain embodiments the selected subject is neither nonhyperlipidemic nor receiving treatment for hyperlipidemia.

In certain embodiments, the selected subject is apparently healthy. "Apparently healthy," as used herein, means individuals who have not previously had an acute, adverse cardiovascular event such as a myocardial infarction (i.e., individuals who are not at an elevated risk of a second adverse cardiovascular event due to a primary adverse cardiovascular event). Apparently healthy individuals also do not otherwise exhibit symptoms of disease.

In certain embodiments, the selected subject has previously suffered an acute adverse cardiovascular event such as a myocardial infarction, stroke, angina pectoris and/or peripheral arteriovascular disease. In one embodiment, the selected subject has previously suffered an acute adverse cardiovascular event such as a myocardial infarction, stroke, angina pectoris and/or peripheral arteriovascular disease, but is nonhyperlipidemic. In one embodiment, the selected subject has previously suffered an acute adverse cardiovascular event such as a myocardial infarction, stroke, angina pectoris and/or peripheral arteriovascular disease, but is neither nonhyperlipidemic nor receiving treatment for hyperlipidemia.

In certain embodiments, the selected subject has a disease or disorder selected from the group consisting of type I diabetes mellitus, type II diabetes mellitus, Kawasaki disease, chronic inflammatory disease, and hypertension. In one embodiment, the selected subject has a disease or disorder selected from the group consisting of type I diabetes mellitus, type II diabetes mellitus, Kawasaki disease, chronic inflammatory disease, and hypertension, but is nonhyperlipidemic. In one embodiment, the selected subject has a disease or disorder selected from the group consisting of type I diabetes mellitus, type II diabetes mellitus, Kawasaki disease, chronic inflammatory disease, and hypertension, but is neither nonhyperlipidemic nor receiving treatment for hyperlipidemia.

In certain embodiments, the selected subject has elevated levels of an inflammatory marker. In one embodiment, the selected subject has elevated levels of an inflammatory marker, but is nonhyperlipidemic. Any marker of systemic inflammation can be utilized for the purposes of the present invention. Suitable inflammatory markers include, without limitation, C-reactive protein (see e.g., US Patent Number 7,964,614, which is incorporated by reference herein in its entirety), cytokines (e.g., II-6, IL-8, and/or IL-17), and cellular adhesion molecules (e.g., ICAM-1, ICAM-3, BL-CAM, LFA-2, VCAM-1, NCAM, and PECAM).

The level of an inflammatory marker can be obtained by any art-recognized assay. Typically, the level is determined by measuring the level of the marker in a body fluid, for example, blood, lymph, saliva, urine and the like. The level can be determined by ELISA, or immunoassays or other conventional techniques for determining the presence of the marker. To determine if levels of the inflammatory marker are elevated, the level of the marker measured in a subject can be compared to a suitable control (e.g., a predetermined value and/or a value obtained from a matched healthy subject).

In one embodiment, present invention provides a pharmaceutical composition comprising a PCSK9 inhibitor for use in the inhibition of atherosclerotic plaque formation in a subject, as well as in treating or inhibiting progression of atherosclerosis in a subject.

The present invention also provides uses of a pharmaceutical composition comprising a PCSK9 inhibitor for the manufacture of a medicament for any of the methods described herein, including inhibition of atherosclerotic plaque formation, and treating or inhibiting progression of atherosclerosis in a subject.

### PCSK9 Inhibitors

In certain aspects, the methods of the present invention comprise administering to a subject a therapeutic composition comprising a PCSK9 inhibitor.

As used herein, a "PCSK9 inhibitor" is any agent which binds to or interacts with human PCSK9 and inhibits the normal biological function of PCSK9 *in vitro* or *in vivo.* Non-limiting examples of categories of PCSK9 inhibitors include small molecule PCSK9 antagonists, antagonistic nucleic acid molecules (e.g., RNAi molecules) peptide-based PCSK9 antagonists (*e.g*., "peptibody" molecules), and antibodies or antigen-binding fragments of antibodies that specifically bind human PCSK9.

As used herein, the term "proprotein convertase subtilisin/kexin type 9" or "PCSK9" refers to PCSK9 having the nucleic acid sequence shown in SEQ ID NO:754 and the amino acid sequence of SEQ ID NO:755, or a biologically active fragment thereof.

In certain embodiments, administration of the PCSK9 inhibitor reduces atherosclerotic plaque formation in the subject (e.g., a human subject) by at least 10% (e.g., 10% 20%, 30%, 40%, 50%, 60%, 70%, 80%, 90%, or 100%) relative to atherosclerotic plaque formation in an untreated subject.

In certain embodiments, the PCSK9 inhibitor is an antibody or antigen-binding protein that specifically bind to PCSK9. As used herein, the term "antibody" refers to immunoglobulin molecules comprising four polypeptide chains, two heavy (H) chains and two light (L) chains inter-connected by disulfide bonds, as well as multimers thereof (e.g., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules and antigen-binding proteins. The terms "antigen-binding portion" of an antibody, "antigen-binding fragment" of an antibody, "antigen-binding protein," and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments or antigen-binding proteins include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains, are also encompassed within the expressions "antigen-binding fragment" and 'antigen-binding proteins" as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H}I ; (ii) V_{H} - C_{H}2; (iii) V_{H} -C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3, (xi) V_{L}-C_{H}I-C_{H}2; (xii) V_{L}-C_{H}I-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (e.g., 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present invention may comprise a homo-dimer or hetero-dimer (or other multimer) of any of the variable and constant domain configurations listed above in non-covalent association with one another and/or with one or more monomeric V_{H} or V_{L} domain (e.g., by disulfide bond(s)).

As with full antibody molecules, antigen-binding fragments may be monospecific or multispecific (e.g., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody of the present invention using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies of the invention may nonetheless include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and V_{L} regions of the recombinant antibodies are sequences that, while derived from and related to human germline VH and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via interchain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (see e.g., Angal et al. (1993) Molecular Immunology 30:105) to levels typically observed using a human IgG1 hinge. The instant invention encompasses antibodies having one or more mutations in the hinge, CH2 or CH3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding protein forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present invention, includes antibodies that bind PCSK9 or portion thereof with a KD of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9 can, however, have cross-reactivity to other antigens, such as PCSK9 molecules from other (non-human) species.

Exemplary, non-limiting, PCSK9 inhibitors are set forth herein and include for example, inhibitors described in US Patent Application Publication Nos. US20130115223, US20130071405, US20130064834, US20130064825, US20120122954, US20120015435, US20110313024, US 20110015252, US20110230542, US20110009628, and US Serial Number 61/682,349, which are each incorporated herein by reference in their entireties. In some embodiments, the PCSK9 inhibitor is an antibody, including an antibody having VH/VL sequences of Ab "LGT209" as described in International Publication No. WO2011/072263; an antibody having VH/VL sequences of Ab "L1L3" as described in International Publication No. WO2010/029513; an antibody having VH/VL sequences of Ab "5L1721 H23_6L3" as described in International Publication No. WO2011/111007; an antibody having VH/VL sequences of Ab "5L1721H23_6L3H3" as described in International Publication No. WO2011/111007; an antibody having VH/VL sequences of Ab "31H4" as described in International Publication No. WO2009/026558; an antibody having VH/VL sequences of Ab "1B20" as described in US Patent Application Publication No. US 2009/0232795; an antibody having VH/VL sequences of Ab "21 B12" described in US Patent Application Publication No. US 2009/0142352; an antibody having VH/VL sequences of Ab "508.20.28" as described in US Patent Application Publication No. US 2012/0195910; or an antibody having VH/VL sequences of Ab "508.20.33" as described in US Patent Application Publication No. US 2012/0195910.

Anti-PCSK9 antibodies useful in the methods and compositions of the present invention may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present invention includes methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain embodiments, all of the framework and/or CDR residues within the VH and/or V_{L} domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (i.e., a germline sequence that is different from the germline sequence from which the antibody was originally derived). Furthermore, the antibodies of the present invention may contain any combination of two or more germline mutations within the framework and/or CDR regions, e.g., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The present invention also includes methods involving the use of anti-PCSK9 antibodies comprising variants of any of the HCVR, LCVR, and/or CDR amino acid sequences disclosed herein having one or more conservative substitutions. For example, the present invention includes the use of anti-PCSK9 antibodies having HCVR, LCVR, and/or CDR amino acid sequences with, e.g., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore ^{™} system (Biacore Life Sciences division of GE Healthcare, Piscataway, NJ).

The term "K_{D}", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain embodiments, the anti-PCSK9 antibody used in the methods of the present invention is an antibody with pH-dependent binding characteristics. As used herein, the expression "pH-dependent binding" means that the antibody or antigen-binding protein exhibits "reduced binding to PCSK9 at acidic pH as compared to neutral pH" (for purposes of the present disclosure, both expressions may be used interchangeably). For the example, antibodies "with pH-dependent binding characteristics" includes antibodies and antigen-binding fragments thereof that bind PCSK9 with higher affinity at neutral pH than at acidic pH. In certain embodiments, the antibodies and antigen-binding fragments of the present invention bind PCSK9 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

According to this aspect of the invention, the anti-PCSK9 antibodies with pH-dependent binding characteristics may possess one or more amino acid variations relative to the parental anti-PCSK9 antibody. For example, an anti-PCSK9 antibody with pH-dependent binding characteristics may contain one or more histidine substitutions or insertions, *e.g*., in one or more CDRs of a parental anti-PCSK9 antibody. Thus, according to certain embodiments of the present invention, methods are provided comprising administering an anti-PCSK9 antibody which comprises CDR amino acid sequences (*e.g*., heavy and light chain CDRs) which are identical to the CDR amino acid sequences of a parental anti-PCSK9 antibody, except for the substitution of one or more amino acids of one or more CDRs of the parental antibody with a histidine residue. The anti-PCSK9 antibodies with pH-dependent binding may possess, e.g., 1, 2, 3, 4, 5, 6, 7, 8, 9, or more histidine substitutions, either within a single CDR of a parental antibody or distributed throughout multiple (*e.g*., 2, 3, 4, 5, or 6) CDRs of a parental anti-PCSK9 antibody. For example, the present invention includes the use of anti-PCSK9 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-PCSK9 antibody.

As used herein, the expression "acidic pH" means a pH of 6.0 or less (*e.g*., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

### Preparation of Human Antibodies

Methods for generating human antibodies in transgenic mice are known in the art. Any such known methods can be used in the context of the present invention to make human antibodies that specifically bind to human PCSK9.

Using VELOCIMMUNE™ technology (see, for example, US 6,596,541, Regeneron Pharmaceuticals) or any other known method for generating monoclonal antibodies, high affinity chimeric antibodies to PCSK9 are initially isolated having a human variable region and a mouse constant region. The VELOCIMMUNE® technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE® mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc., using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies that can be used in the methods of the present invention possess high affinities, as described above, when measured by binding to antigen either immobilized on solid phase or in solution phase. The mouse constant regions are replaced with desired human constant regions to generate the fully human antibodies of the invention. While the constant region selected may vary according to specific use, high affinity antigen-binding and target specificity characteristics reside in the variable region.

Specific examples of human antibodies or antigen-binding fragments of antibodies that specifically bind PCSK9 which can be used in the context of the methods of the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs: 2, 18, 22, 26, 42, 46, 50, 66, 70, 74, 90, 94, 98, 114, 118, 122, 138, 142, 146, 162, 166, 170, 186, 190, 194, 210, 214, 218, 234, 238, 242, 258, 262, 266, 282, 286, 290, 306, 310, 314, 330, 334, 338, 354, 358, 362, 378, 382, 386, 402, 406, 410, 426, 430, 434, 450, 454, 458, 474, 478, 482, 498, 502, 506, 522, 526, 530, 546, 550, 554, 570, 574, 578, 594, 598, 602, 618, 622, 626, 642, 646, 650, 666, 670, 674, 690, 694, 698, 714, 718, 722, 738 and 742, or a substantially similar sequence thereof having at least 90%, at least 95%, at least 98% or at least 99% sequence identity. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs: 10, 20, 24, 34, 44, 48, 58, 68, 72, 82, 92, 96, 106, 116, 120, 130, 140, 144, 154, 164, 168, 178, 188, 192, 202, 212, 216, 226, 236, 240, 250, 260, 264, 274, 284, 288, 298, 308, 312, 322, 332, 336, 346, 356, 360, 370, 380, 384, 394, 404, 408, 418, 428, 432, 442, 452, 456, 466, 476, 480, 490, 500, 504, 514, 524, 528, 538, 548, 552, 562, 572, 576, 586, 596, 600, 610, 620, 624, 634, 644, 648, 658, 668, 672, 682, 692, 696, 706, 716, 720, 730, 740 and 744, or a substantially similar sequence thereof having at least 90%, at least 95%, at least 98% or at least 99% sequence identity.

In certain embodiments of the present invention, the antibody or antigen-binding protein comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744.

In certain embodiments of the present invention, the anti-PCSK9 antibody, or antigen-binding protein, that can be used in the methods of the present invention has HCDR1/HCDR2/HCDR3/LCDR1/LCDR2/LCDR3 amino acid sequences selected from SEQ ID NOs: 76/78/80/84/86/88 (mAb316P) and 220/222/224/228/230/232 (mAb300N) (See US Patent App. Publ No. 2010/0166768) and 220/222/224/228/230/232, wherein SEQ ID NOL228 comprises a substitution of histidine for leucine at amino acid residue 30 (L30H).

In certain embodiments of the present invention, the antibody or antigen-binding protein comprises HCVR/LCVR amino acid sequence pairs selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744. In certain exemplary embodiments, the antibody or antigen-binding protein comprises an HCVR amino acid sequence of SEQ ID NO:90 and an LCVR amino acid sequence of SEQ ID NO:92. In certain exemplary embodiments, the antibody or antigen-binding protein comprises an HCVR amino acid sequence of SEQ ID NO:218 and an LCVR amino acid sequence of SEQ ID NO:226. In certain exemplary embodiments, the antibody or antigen-binding protein comprises an HCVR amino acid sequence of SEQ ID NO:218 and an LCVR amino acid sequence of SEQ ID NO:226 comprising a substitution of histidine for leucine at amino acid residue 30 (L30H).

In certain embodiments, the antibody or antigen-binding protein exhibits one or more or the following properties when administered by weekly subcutaneous injection to an APOE*3Leiden.CETP mouse:
a. reduces total cholesterol levels relative to untreated controls by about 37% when administered at 3 mg/kg;
b. reduces total cholesterol levels relative to untreated controls by about 46% when administered at 10 mg/kg;
c. reduces total cholesterol levels relative to untreated controls by about 48% when administered at 3 mg/kg in combination with 3.6 mg/kg/day atorvastin;
d. reduces total cholesterol levels relative to untreated controls by about 58% when administered at 10 mg/kg in combination with 3.6 mg/kg/day atorvastin;
e. reduces total cholesterol levels by about 36% when administered at 3 mg/kg in combination with 3.6 mg/kg/day atorvastin, relative to treatment with 3.6 mg/kg/day atorvastin alone;
f. reduces total cholesterol levels by about 48% when administered at 10 mg/kg in combination with 3.6 mg/kg/day atorvastin, relative to treatment with 3.6 mg/kg/day atorvastin alone;
g. reduces triglyceride levels relative to untreated controls by about 33% when administered at 3 mg/kg;
h. reduces triglyceride levels relative to untreated controls by about 36% when administered at 10 mg/kg;
i. reduces triglyceride levels by about 40% when administered at 3 mg/kg in combination with 3.6 mg/kg/day atorvastin, relative to treatment with 3.6 mg/kg/day atorvastin;
j. reduces triglyceride levels by about 51% when administered at 10 mg/kg in combination with 3.6 mg/kg/day atorvastin, relative to treatment with 3.6 mg/kg/day atorvastin alone;
k. increases hepatic LDLR expression relative to untreated controls by about 88% when administered at 3 mg/kg;
I. increases hepatic LDLR expression relative to untreated controls by about 178% when administered at 10 mg/kg;
m. increases hepatic LDLR expression by about 71% when administered at 3 mg/kg in combination with 3.6 mg/kg/day atorvastin, relative to treatment with 3.6 mg/kg/day atorvastin alone;
n. increases hepatic LDLR expression by about 140% when administered at 10 mg/kg in combination with 3.6 mg/kg/day atorvastin, relative to treatment with 3.6 mg/kg/day atorvastin alone;
o. decreases atherosclerotic lesion size relative to untreated controls by about 70% when administered at 3 mg/kg;
p. decreases atherosclerotic lesion size relative to untreated controls by about 87% when administered at 10 mg/kg;
q. decreases atherosclerotic lesion size relative to untreated controls by about 88% when administered at 3 mg/kg in combination with 3.6 mg/kg/day atorvastin;
r. decreases atherosclerotic lesion size relative to untreated controls by about 98% when administered at 10 mg/kg in combination with 3.6 mg/kg/day atorvastin;
s. decreases atherosclerotic lesion size relative by about 82% when administered at 3 mg/kg in combination with 3.6 mg/kg/day atorvastin, relative to treatment with 3.6 mg/kg/day atorvastin alone;
t. decreases atherosclerotic lesion size relative by about 97% when administered at 10 mg/kg in combination with 3.6 mg/kg/day atorvastin, relative to treatment with 3.6 mg/kg/day atorvastin alone;
u. reduces triglyceride levels by about 72% when administered at 3 mg/kg, relative to treatment with 3.6 mg/kg/day atorvastatin alone;
v. reduces triglyceride levels by about 79% when administered at 10 mg/kg, relative to treatment with 3.6 mg/kg/day atorvastatin alone;
w. reduces total cholesterol levels by about 22% when administered at 3 mg/kg, relative to treatment with 3.6 mg/kg/day atorvastatin alone;
x. reduces total cholesterol levels by about 34% when administered at 10 mg/kg, relative to treatment with 3.6 mg/kg/day atorvastatin alone;
y. increases the percentage of undiseased aortic segments by about 236% when administered at 3 mg/kg, relative to untreated control;
z. increases the percentage of undiseased aortic segments by about 549% when administered at 10 mg/kg, relative to untreated control;
aa. increases the percentage of undiseased aortic segments by about 607% when administered at 3 mg/kg in combination with 3.6 mg/kg/day atorvastatin, relative to control; and
bb. increases the percentage of undiseased aortic segments by about 1118% when administered at 3 mg/kg in combination with 3.6 mg/kg/day atorvastatin, relative to control.

### Pharmaceutical Compositions and Methods of Administration

The present invention includes methods which comprise administering a PCSK9 inhibitor to a subject, wherein the PCSK9 inhibitor is contained within a pharmaceutical composition. The pharmaceutical compositions of the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition of the invention, e.g., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, inhalation, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the present invention can be delivered subcutaneously or intravenously with a standard needle and syringe. In addition, pen delivery devices and autoinjector delivery devices readily have applications in delivering a pharmaceutical composition of the present invention. Such delivery devices can be reusable or disposable, and can be adapted to administer a variable dose or a fixed dose.. A reusable delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The delivery device can then be reused. In a disposable delivery device, there is no replaceable cartridge. Rather, the disposable delivery device comes prefilled with the pharmaceutical composition held in a reservoir within the device. Once the reservoir is emptied of the pharmaceutical composition, the entire device is discarded. A delivery device adapted for variable dosing may include a mechanism for setting a dose within a range of dose volumes (in some cases, the range may be limited to a value less than a total volume contained in the cartridge). A delivery device adapted for fixed dosing may include a mechanism which delivers the total volume of the cartridge when the delivery device it actuated. In such cases, the cartridge may be a pre-filled syringe.

Numerous delivery devices have applications in the delivery of a pharmaceutical composition of the present invention. Examples include, but are not limited to AUTOPEN ^{™} (Owen Mumford, Inc., Woodstock, UK), DISETRONICTM pen (Disetronic Medical Systems, Bergdorf, Switzerland), HUMALOG MIX 75/25¹m pen, HUMALOGTM pen, HUMALIN 70/30TM pen (Eli Lilly and Co., Indianapolis, IN), NOVOPENTM I, II and III (Novo Nordisk, Copenhagen, Denmark), NOVOPEN JUNIORTM (Novo Nordisk, Copenhagen, Denmark), BDTM pen (Becton Dickinson, Franklin Lakes, NJ), OPTIPENTM, OPTIPEN PROTM, OPTIPEN STARLETTM, and OPTICLIKTM (sanofi-aventis, Frankfurt, Germany), to name only a few. Examples of disposable pen delivery devices having applications in subcutaneous delivery of a pharmaceutical composition of the present invention include, but are not limited to the SOLOSTARTM pen (sanofi-aventis), the FLEXPEN ^{™} (Novo Nordisk), and the KWIKPEN ^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL), to name only a few. In some embodiments, the reusable pen or autoinjector delivers a fixed dose. In other embodiments, the reusable pen or autoinjector can deliver a variable dose. In different embodiments, the reusable pen or autoinjector can deliver a single dose or multiple doses.

In certain embodiments, the pharmaceutical composition is delivered in a controlled release system. In certain embodiments, a pump may be used (see Langer, supra; Sefton, 1987, CRC Crit. Ref. Biomed. Eng. 14:201), including a micropump. In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, e.g., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e.g.*, by dissolving, suspending or emulsifying the antibody or its salt described above *in* a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil], etc. As the oily medium, there are employed, e.g., sesame oil, soybean oil, etc., which may be used in combination with a solubilizing agent such as benzyl benzoate, benzyl alcohol, etc. The injection thus prepared is filled in an appropriate ampoule.

Advantageously, the pharmaceutical compositions for oral or parenteral use described above are prepared into dosage forms in a unit dose suited to fit a dose of the active ingredients. As used herein, "unit dosage form" refers to physically discrete units suitable as single dosages for human and/or animal subjects, each unit containing a predetermined quantity of active material (i.e., a PCSK9 inhibitor) calculated to produce the desired therapeutic effect in association with a pharmaceutical diluent, carrier or vehicle. In some embodiments, the PCSK9 inhibitor is an antibody or antigen-binding protein and the unit dosage form comprises 75 mg, 150 mg, 200 mg, or 300 mg of the antibody or antigen-binding protein. Examples of suitable unit dosage forms for liquid pharmaceutical compositions include applicators such as vials, syringes, including pre-filled syringes and reusable syringes, autoinjectors, including pre-filled autoinjectors and reusable autoinjectors, cartridges, and ampules; other suitable unit dosage forms include tablets, pills, capsules, suppositories, wafers, segregated multiples of any of the foregoing, and other forms as herein described or generally known in the art. The unit dosage form may be hermetically sealed. For unit dosage forms containing the pharmaceutical composition within an applicator, the quantity of the PCSK9 inhibitor may be indicated on the applicator.

The present invention includes includes an article of manufacture or kit comprising (a) one or more unit dosage forms comprising a pharmaceutical composition of present invention, and (b) a container or package. The article of manufacture or kit can further comprise (c) a label or packaging insert with instructions for use. In some embodiments, the article of manufacture can further comprise (d) one or more unit dosage forms of a lipid-modifying therapy (e.g. a blister of tablets comprising as an active ingredient an HMG-CoA reductase inhibitor).

### Dosage and Administration Regimens

The amount of PCSK9 inhibitor (*e.g*., anti-PCSK9 antibody) administered to a subject according to the methods and compositions of the present invention is, generally, a therapeutically effective amount. As used herein, the phrase "therapeutically effective amount" means a dose of PCSK9 inhibitor that results in a detectable reduction in atherosclerotic lesions. For example, "therapeutically effective amount" of a PCSK9 inhibitor includes, e.g., an amount of PCSK9 inhibitor that causes a reduction of at least 2%, 5%, 10%, 15%, 20%, 25%, 30%, 40%, 50%, 60%, 70%, 80%, 90% or more in sclerotic lesion area or lesion severity when administered to a human patient, e.g., as illustrated in the Examples herein. Alternatively, animal models can be used to establish whether a particular amount of a candidate PCSK9 inhibitor is a therapeutically effective amount.

In the case of an anti-PCSK9 antibody, a therapeutically effective amount can be from about 0.05 mg to about 600 mg, e.g., about 0.05 mg, about 0.1 mg, about 1.0 mg, about 1.5 mg, about 2.0 mg, about 3mg, about 5 mg, about 10 mg, about 15 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody.

In certain embodiments, the anti-PCSK9 antibody is administered to a subject at a dose of 75 mg. In certain embodiments, the anti-PCSK9 antibody is administered to a subject at a dose of 150 mg. In certain embodiments, the anti-PCSK9 antibody is administered to a subject at a dose of 300 mg.

The amount of anti-PCSK9 antibody contained within the individual doses may be expressed in terms of milligrams of antibody per kilogram of patient body weight (i.e., mg/kg). For example, the anti-PCSK9 antibody may be administered to a patient at a dose of about 0.0001 to about 10 mg/kg of patient body weight.

According to certain embodiments of the present invention, multiple doses of a PCSK9 inhibitor may be administered to a subject over a defined time course. The methods according to this aspect of the invention comprise sequentially administering to a subject multiple doses of a PCSK9 inhibitor. As used herein, "sequentially administering" means that each dose of PCSK9 inhibitor is administered to the subject at a different point in time, e.g., on different days separated by a predetermined interval (e.g., hours, days, weeks or months). The present invention includes methods which comprise sequentially administering to the patient a single initial dose of a PCSK9 inhibitor, followed by one or more secondary doses of the PCSK9 inhibitor, and optionally followed by one or more tertiary doses of the PCSK9 inhibitor.

The terms "initial dose," "secondary doses," and "tertiary doses," refer to the temporal sequence of administration of the PCSK9 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. In certain embodiments, however, the amount of PCSK9 inhibitor contained in the initial, secondary and/or tertiary doses will vary from one another (e.g., adjusted up or down as appropriate) during the course of treatment. The initial, secondary, and tertiary doses may all contain the same amount of PCSK9 inhibitor.

In certain embodiments, each secondary and/or tertiary dose is administered 1 to 30 (*e.g*., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more) days after the immediately preceding dose. The phrase "the immediately preceding dose," as used herein, means, in a sequence of multiple administrations, the dose of PCSK9 inhibitor which is administered to a patient prior to the administration of the very next dose in the sequence with no intervening doses.

In certain embodiments, the methods comprise administering to a patient any number of secondary and/or tertiary doses of a PCSK9 inhibitor. For example, in certain embodiments, only a single secondary dose is administered to the patient. In other embodiments, two or more (*e.g*., 2, 3, 4, 5, 6, 7, 8, or more) secondary doses are administered to the patient. Likewise, in certain embodiments, only a single tertiary dose is administered to the patient. In other embodiments, two or more (e.g., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 29 days after the immediately preceding dose. Similarly, in embodiments involving multiple tertiary doses, each tertiary dose may be administered at the same frequency as the other tertiary doses. For example, each tertiary dose may be administered to the patient 1 to 60 days after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

In certain embodiments, the anti-PCSK9 antibody is administered to a subject at an initial dose of at about 75 mg every two weeks.

In additional embodiments, the antibody is administered to a subject at an initial dose of about 150 mg every two weeks.

In one embodiment, an initial dose is administered during an initial dosing period, after which the subject's LDL-C value is monitored determine if the initial dose should be changed to a secondary dose, by measuring the LDL-C value to determine if it is at or below a target LDL-C value. For example, a subject may begin treatment with an initial dose of 75 mg of the antibody, with a target LDL-C value of 100 milligrams per deciliter (mg/dL). If the subject's LDL-C reaches a target LDL-C value at the end of the initial dosing period, then the subject continues treatment on the initial dose, but changes to a secondary dose if that target value is not reached. In one embodiment, the initial dose is about 75 mg, administered every two weeks, the target LDL-C value is 100 mg/dL, and the secondary dose, if necessary, is 150 mg every two weeks. For example, the subject may be first monitored after about 8 weeks of treatment, and if their LDL-C value is above 100 mg/dL, the subject may be uptitrated to a secondary dose of 150 mg, administered every two weeks. Monitoring may continue throughout the treatment period. In another embodiment, the initial dose is 75 mg of antibody every two weeks, the target LDL-C value is 70 mg/dL, and the secondary dose is 150 mg every two weeks if the target LDL-C value is not reached.

### Prior Therapies and Combination Therapies

The methods of the present invention, according to certain embodiments, comprise administering a pharmaceutical composition comprising an anti-PCSK9 antibody to a subject who is on a therapeutic regimen for the treatment of hypercholesterolemia and/or atherosclerosis at the time of, or just prior to, administration of the pharmaceutical composition of the invention. For example, a patient who has previously been diagnosed with hypercholesterolemia and/or atherosclerosis may have been prescribed and is taking a stable therapeutic regimen of another lipid modifying therapy prior to and/or concurrent with administration of a pharmaceutical composition comprising an anti-PCSK9 antibody. In other embodiments, the subject has not been previously treated with a lipid modifying therapy.

The methods of the present invention, according to certain embodiments, comprise administering as a monotherapy a pharmaceutical composition comprising an anti-PCSK9 antibody to a subject, in the absence of any concurrent lipid modifying therapy.

The methods of the present invention, according to certain embodiments, also comprise administering a pharmaceutical composition comprising an anti-CPSK9 inhibitor to a subject in combination with another lipid modifying therapy

As used herein, lipid modifying therapies include, for example, (1) agents which induce a cellular depletion of cholesterol synthesis by inhibiting 3-hydroxy-3-methylglutaryl (HMG)-coenzyme A (CoA) reductase, such as a statin (e.g., cerivastatin, atorvastatin, simvastatin, pitavastatin, rosuvastatin, fluvastatin, lovastatin, pravastatin, etc.); (2) agents which inhibit cholesterol uptake and or bile acid re-absorption (such as ezetimibe); (3) agents which increase lipoprotein catabolism (such as niacin); and/or (4) activators of the LXR transcription factor that plays a role in cholesterol elimination such as 22-hydroxycholesterol. Lipid modifying therapies also include fixed combinations of therapeutic agents such as ezetimibe plus simvastatin; a statin with a bile resin (e.g., cholestyramine, colestipol, colesevelam); niacin plus a statin (e.g., niacin with lovastatin); or with other lipid lowering agents such as omega-3-fatty acid ethyl esters (for example, omacor).

### EXAMPLES

The following examples are put forth so as to provide those of ordinary skill in the art with a complete disclosure and description of how to make and use the methods and compositions of the invention, and are not intended to limit the scope of what the inventors regard as their invention.

### Example 1: Generation of Human Antibodies to Human PCSK9

Human anti-PCSK9 antibodies were generated as described in US Patent No. 8,062,640. The exemplary PCSK9 inhibitor used in the following Example is the human anti-PCSK9 antibody designated "mAb316P," also known here as "Alirocumab." mAb316P has the following amino acid sequence characteristics: heavy chain variable region (HCVR) comprising SEQ ID NO:90; light chain variable domain (LCVR) comprising SEQ ID NO:92; heavy chain complementarity determining region 1 (HCDR1) comprising SEQ ID NO:76; HCDR2 comprising SEQ ID NO:78; HCDR3 comprising SEQ ID NO:80; light chain complementarity determining region 1 (LCDR1) comprising SEQ ID NO:84; LCDR2 comprising SEQ ID NO:86; and LCDR3 comprising SEQ ID NO:88.

### Example 2: Attainment of Low-Density Lipoprotein Cholesterol Goals in Patients at High Cardiovascular Risk: Results from a Managed Care Population Study

Background: US guidelines support LDL-C goals based on patient's cardiovascular (CV) risk profile, however, little is known regarding real-world patterns of LDL-C goal attainment within specific high CV risk conditions.

Methods: Patients from the OptumInsight IMPACT Database (a large US multi-payer claims database) with an LDL-C measurement during July 2011 to June 2012 and high CV risk conditions were identified. Most recent LDL-C measurement was defined as the index date and high CV risk conditions were identified hierarchically during the pre-index period as follows: recent acute coronary syndrome (ACS, within 6 months pre-index date), coronary events (myocardial infarction, hospitalization for unstable angina, coronary revascularization), stroke, and peripheral vascular disease (PVD).

Results: In total, 110,739 patients met the inclusion criterion. Median (IQR) age was 59 (53 to 65) years, 53.7% were male, and median (IQR) LDL-C was 116 (92 to 143) mg/dL. As of index date, 2.7% had a recent ACS, while 42.1%, 9.2%, and 46.0% had evidence of coronary events, stroke, and PVD, respectively. The following table (Table 1) represents a summary distribution of patients by LDL-C levels as of index date for different high CV risk conditions.

**Table 1: Breakdown of patients by LDL-C level (mg/dL) and risk condition.**

| High CV Risk Condition (% Patients; N) | % Patients by LDL-C Level (mg/dL) | | | | | |
|---|---|---|---|---|---|---|
| | <70 | ≥70 to <100 | ≥100 to <130 | ≥130 to <160 | ≥160 | Any |
| Recent ACS (2.7%; 2,966) | 17.2% | 27.4% | 26.7% | 16.5% | 12.1% | 100.0% |
| Coronary Events (42.1%; 46,616) | 9.5% | 25.1% | 30.3% | 21.4% | 13.8% | 100.0% |
| Stroke (9.2%; 10,240) | 8.0% | 23.8% | 30.4% | 23.5% | 14.3% | 100.0% |
| PVD (46.0%; 50,917) | 6.9% | 23.0% | 31.8% | 23.8% | 14.5% | 100.0% |
| Overall (100%; 110,739) | 8.3% | 24.0% | 30.9% | 22.6% | 14.1% | 100.0% |

Conclusions: In a large, contemporary cohort of high CV risk patients, few achieved LDL-C goals of <70 mg/dL (optional goal for very high CV risk) or <100 mg/dL. Although LDL-C goal achievement improved marginally in patients with conditions signifying higher CV risk, with highest achievement in patients with recent ACS, the majority of these patients were still above LDL-C goals. These data show that there is a need for ongoing efforts to address gaps in LDL-C goal attainment and improving CV outcomes in high-risk patients.

### Example 3: Low-Density Lipoprotein Cholesterol Goal Attainment and Lipid-Lowering Therapy in a High Cardiovascular Risk Managed Care Population

Background: While US guidelines support statins as first line therapy to reduce LDL-C, little is known regarding real-world patterns of LDL-C goal attainment with statins and other lipid-lowering therapies (LLTs).

Methods: Patients from the OptumInsight IMPACT Database (a large US multi-payer claims database) with an LDL-C measurement during July 2011 to June 2012 and high risk CV conditions (coronary events (myocardial infarction, hospitalization for unstable angina, coronary revascularization), stroke, and peripheral vascular disease) were identified. LLT prescription was assessed as of most recent LDL-C measurement (index date) and categorized as high-potency statin (atorvastatin 40/80 mg, rosuvastatin 20/40 mg, simvastatin 80 mg), standard-potency statin (other statins), non-statin LLT (ezetimibe, niacin, fibrates, bile acid sequestrants), and no LLT.

Results: In total, 110,739 patients met the inclusion criterion. Median (IQR) age was 59 (53 to 65) years, 53.7% were male, and median (IQR) LDL-C was 116 (92 to 143) mg/dL. As of index date, 10.8% were on high-potency statin, 26.9% were on standard-potency statin, 5.3% were on non-statin LLT, and 57.0% were not on any LLT. The following table (Table 2) represents a summary distribution of patients by LDL-C levels as of index date for different LLT types.

**Table 2: Breakdown of patients by LDL-C level (mg/dL) and LLT type.**

| LLT Type (% Patients; N) | % Patients by LDL-C Level (mg/dL) | | | | | |
|---|---|---|---|---|---|---|
| | <70 | ≥70 to <100 | ≥100 to <130 | ≥130 to <160 | ≥160 | Any |
| High-Potency Statin (10.8%; 12,014) | 15.2% | 33.7% | 26.0% | 13.7% | 11.4% | 100.0% |
| Standard-Potency Statin (26.9%; 29,734) | 11.2% | 30.1% | 28.5% | 18.2% | 12.0% | 100.0% |
| Non-Statin LLT (5.3%; 5,921) | 12.1% | 27.6% | 29.8% | 19.0% | 11.5% | 100.0% |
| No LLT (57.0%; 63,070) | 5.3% | 19.0% | 33.1% | 26.7% | 15.9% | 100.0% |
| Overall (100%; 110,739) | 8.3% | 24.0% | 30.9% | 22.6% | 14.1% | 100.0% |

Conclusions: In this large, contemporary population of patients at high CV risk, few achieved LDL-C goals of <70 mg/dL (optional goal for very high CV risk) or <100 mg/dL. Further, many other patients were not at LDL-C goal despite receiving a high-potency statin. These data show that there is a need for ongoing efforts to improve adherence to LLT as well as new therapies to improve goal attainment and CV outcomes.

### Example 4: Monoclonal Antibody to PCSK-9, Alirocumab Dose-Dependently Decreases Atherosclerosis, Induces a More Stable Plaque Phenotype and Enhances the Effects of Atorvastatin in APOE*3Leiden.CETP Transgenic Mice

### Background

The aim of this study was to investigate the effects of two dosages of alirocumab, alone and in combination with atorvastatin, on plasma lipids, atherosclerosis development, and lesion composition in APOE*3Leiden.CETP mice. This is a well-established model for hyperlipidemia and atherosclerosis with all features of familial dysbetalipoproteinemia (FD) in humans, which is characterized by accumulation of remnant lipoproteins and an increased very LDL (VLDL) cholesterol to LDL-C ratio. APOE*3Leiden mice have an impaired clearance of (V)LDL and increased TG levels, and are thereby mimicking the slow clearance observed in humans, in contrast to normal wild-type mice, which have a very rapid clearance of apoB-containing lipoproteins. The lipoprotein profile in APOE*3Leiden.CETP mice reflects that of FD patients with a similar response to lipid-modifying therapies, including statins, fibrates, niacin, and cholesteryl ester transfer protein (CETP) inhibitors. This is illustrated by a comparable reduction in cholesterol in all apoB-containing lipoprotein subfractions with statin treatment. It was hypothesized that alirocumab alone could reduce progression of atherosclerosis and add to the atheroprotective effects of atorvastatin. Inhibition of atherosclerosis by atorvastatin in APOE*3Leiden.CETP mice has been observed previously.

### Methods

### i) Animals

Ninety female APOE*3Leiden.CETP transgenic mice (9 to 13 weeks of age), expressing human CETP under control of its natural flanking regions, were used. During the study, mice were housed under standard conditions with a 12-hour light-dark cycle and had free access to food and water. Animal experiments were approved by the Institutional Animal Care and Use Committee of The Netherlands Organization for Applied Research.

### ii) Experimental Design

Mice received a semi-synthetic cholesterol-rich diet, containing 15% (w/w) cacao butter and 0.15% cholesterol (Western-type diet [WTD]; Hope Farms, Woerden, The Netherlands) for a run-in period of 3 weeks to increase plasma total cholesterol (TC) levels up to -15 mmol/L. Body weight (BW) and food intake were monitored regularly during the study. After matching based on BW, TC, plasma TG, and age, mice (n=15 per group) received a WTD alone or were treated with two dosages of alirocumab (3 or 10 mg/kg) alone or in combination with atorvastatin (3.6 mg/kg/d) for 18 weeks, and an arm with atorvastatin alone was added. Alirocumab was administered via weekly subcutaneous injections and atorvastatin was added to the diet. The dose of atorvastatin was calculated in order to attempt a TC reduction of about 20% to 30%. At the end of the treatment period, all animals were sacrificed by CO₂ inhalation. Livers and hearts were isolated to assess hepatic LDLR protein levels, lipid content, atherosclerosis development, and plaque composition.

### iii) Plasma Lipids, Lipoprotein Analysis, and Measurement of Alirocumab Levels

Plasma was isolated from blood collected in ethylenediaminetetraacetic acid (EDTA)-coated cups via tail vein bleeding after a 4-hour fast every 2 to 4 weeks. Plasma TC and TG were determined using enzymatic kits according to the manufacturer's protocols (cat. no. 1458216 and cat. no. 1488872, respectively; Roche/Hitachi) and average plasma TC and TG levels were calculated. Lipoprotein profiles for TC were measured after lipoprotein separation by fast protein liquid chromatography (FPLC) after 4, 12, and 18 weeks of treatment. Alirocumab levels were measured by a human Fc enzyme-linked immunosorbent assay.

### iv) Hepatic LDLR Protein Levels

Liver tissues were homogenized in lysis buffer (50 mM Tris-HCL [pH=7.4], 150 mM NaCl, 0.25% deoxycholic acid, 1% NP-40 [Igepal], 1mM EDTA, protease inhibitor cocktail [complete, Roche], 1 mM PMSF, 1 mM Na3VO4) and then centrifuged at 6500 rpm at 4°C for 30 minutes. Protein concentration in cell lysates was determined by bicinchonic acid protein assay (Thermo Scientific) according to manufacturer's instructions. 50 µg of protein lysates was separated by SDS-PAGE and then transferred to polyvinylidene fluoride membranes (Millipore). Blots were subjected to goat anti-mouse LDLR from R&D Systems and rabbit anti-goat horseradish peroxidase (HRP) from AbD Serotec or mouse anti-α-Tubulin from Sigma and horse anti-mouse HRP from Cell Signaling Technologies (according to the manufacturer's instructions); blots were developed with West Femto Super Signal ECL (Thermo Scientific) and subjected to the Chemi-Doc-it imaging system. Intensities of protein bands were quantified using Image J software.

### v) Histological Assessment of Atherosclerosis

Hearts were isolated, fixed in formalin, and embedded in paraffin. Cross-sections (5 µm each) of the entire aortic root area were stained with hematoxylin-phloxin-saffron. For each mouse, four sections at intervals of 50 µm were used for quantitative and qualitative assessment of the atherosclerotic lesions. To determine atherosclerotic lesion size and severity, the lesions were classified into five categories according to the American Heart Association classification: I) early fatty streak, II) regular fatty streak, III) mild plaque, IV) moderate plaque, and V) severe plaque. Total lesion area and number of lesions per cross-section, as well as the percentage undiseased segments, were calculated. To assess lesion severity as a percentage of all lesions, type I-III lesions were classified as mild lesions, and type IV-V lesions were classified as severe lesions. To determine the total plaque load in the thoracic aorta, perfusion-fixed aortas (from the aortic origin to the diaphragm) were cleaned of extravascular fat, opened longitudinally, pinned en face, and stained for lipids with oil-red O as described previously by Verschuren et al. (Arterioscler Thromb Vasc Biol. 2005;25:161-167). Data were normalized for analyzed surface area and expressed as percentage of the stained area. Photos/images were taken with the Olympus BX51 microscope and lesion areas were measured using Cell D imaging software (Olympus Soft Imaging Solutions).

In the aortic root, lesion composition was determined for the severe lesions (type IV-V) as a percentage of lesion area after immunostaining with mouse anti-human alpha actin (1:800; Monosan, Uden, The Netherlands) for smooth muscle cells (SMC), and rat anti-mouse Mac-3 (1:25; BD Pharmingen, the Netherlands) for macrophages followed by sirius red staining for collagen. Necrotic area and cholesterol clefts, monocyte adhesion to the endothelium, and the calculation of plaque stability index (defined as the ratio of collagen and SMC area as stabilization factors to macrophage and necrotic area as destabilization factors) were determined as previously described by Kuhnast and van der Hoorn et al. (J Hypertens. 2012;30:107-116), Delsing et al. (Circulation. 2001;103:1778-1786), and Kuhnast and Louwe et al. (PLoS One. 2013; 8: e66467). Photos/images of the lesions were taken with the Olympus BX40 microscope with Nuance 2 multispectral imaging system, and stained areas were quantified using Image J software.

### vi) Hepatic Lipid Analysis and Fecal Excretion of Bile Acids and Neutral Sterols

Liver tissue samples were homogenized in phosphate-buffered saline, and the protein content was measured. Lipids were extracted, separated by high-performance thin-layer chromatography on silica gel plates, and analyzed with TINA2.09 software (Raytest Isotopen Messgeräte Straubenhardt, Germany), as previously described by Post et al. (Hepatology. 1999;30:491-500).

Mice were housed at five mice per cage, and feces were collected during two consecutive periods of 72 hours and 48 hours, respectively. Aliquots of lyophilized feces were used for determination of neutral and acidic sterol content by gas-liquid-chromatography, as previously described by Post et al. (Arterioscler Thromb Vasc Biol. 2003;23:892-897).

### vii) Statistical Analysis

Significance of differences between the groups was calculated non-parametrically using a Kruskal-Wallis test for independent samples, followed by a Mann-Whitney U-test for independent samples. Linear regression analyses were used to assess correlations between variables. Since the atherosclerotic lesion area showed a quadratic dependence on plasma cholesterol exposure, it was transformed using square root transformation. IBM SPSS Statistics 20 for Windows (SPSS, Chicago, USA) was used for statistical analyses. All groups were compared to the control group and to the atorvastatin group, and 3 mg/kg alirocumab was compared to 10 mg/kg alirocumab either with or without atorvastatin. Values are presented as means ± SD. P-values <0.05 were considered statistically significant. In the figures, the symbol * is used to compare to the control group, † to compare to the atorvastatin group, and ‡ to compare 3 mg/kg alirocumab to 10 mg/kg alirocumab.

### Results

### i) Alirocumab and Atorvastatin Monotreatment and their Combination Decrease Plasma Total Cholesterol and Triglycerides in APOE*3Leiden.CETP Mice

Circulating alirocumab levels were detected in all groups administered alirocumab and ranged between 5 to 12 µg/mL (3 mg/kg dose) and 12 to 30 µg/mL (10 mg/kg dose) during the 18-week study. The APOE*3Leiden.CETP mice on a cholesterol-containing WTD (control group) reached average plasma TC and TG levels of 16.2 ± 1.8 mmol/L and 2.9 ± 0.6 mmol/L, respectively (Figures 1A and 1 B). Compared to the control, alirocumab decreased average plasma TC (-37%, P<0.001; -46%, P<0.001) and TG (-33%, P<0.001; -36%, P<0.001), and further decreased TC in combination with atorvastatin (-48%, P<0.001; -58%, P<0.001). Compared to atorvastatin, both combination treatments decreased TC (-36%, P<0.001; -48%, P<0.001) and TG (-40%, P<0.001; -51%, P<0.001) to a greater extent than atorvastatin alone. The reductions in TC after alirocumab alone (-14%, P<0.01; 3 mg alirocumab versus 10 mg alirocumab) and in combination with atorvastatin (-19%, P<0.001; 3 mg alirocumab+atorvastatin versus 10 mg alirocumab+atorvastatin) were dose-dependent and sustained during the study. TC reductions after alirocumab (Figure 1C), atorvastatin, and their combination (Figure 1D) were confined to apoB-containing lipoproteins. No effects on BW (gain) and food intake were noted in any treatment groups compared with the control (Table 3).

**Table 3: Safety aspects of alirocumab.**

| | Safety aspects | | |
|---|---|---|---|
| | Plasma ALT (U/L) | Plasma AST (U/L) | Liver weight (gram) |
| Control | 91.6 ± 75.6 | 223.0 ± 189.6 | 1.23 ± 0.25 |
| 3 mg alirocumab | 67.1 ± 42.7 | 189.4 ± 108.7 | 1.18 ± 0.25 |
| 10 mg alirocumab | 153.0 ± 122.6 †† | 426.3 ± 309.2 ** †† | 1.38 ± 0.43 † |
| Atorvastatin | 59.8 ± 24.3 | 217.0 ± 76.5 | 1.05 ± 0.12 |
| 3 mg alirocumab + atorvastatin | 54.5 ± 25.6 | 171.7 ± 66.1 † | 1.02 ± 0.17 * |
| 10 mg alirocumab + atorvastatin | 51.0 ± 23.7 | 157.6 ± 29.2 †† | 0.99 ± 0.10 ** |

| | | | |
|---|---|---|---|
| ALT, alanine transaminase; AST, aspartate transaminase. **P*<0.05, ***P*<0.01 as compared to control; †*P*<0.05, ††*P*<0.01 as compared to atorvastatin | | | |

### ii)Alirocumab, without and with Atorvastatin, Decreases Plasma Lipids by Reducing Low-Density Lipoprotein Receptor Degradation

Hepatic LDLR protein levels were measured to verify whether PCSK9 inhibition by alirocumab decreases plasma lipids by rescuing LDLR degradation (Figure 2). Hepatic LDLR protein levels were increased after alirocumab treatment alone (+80%, P<0.01; +133%, P<0.001) and together with atorvastatin (+98%, P<0.001; +178%, P<0.01). Compared to atorvastatin alone, both the combination treatments increased LDLR protein levels to a greater extent (+71%, P<0.01; +140%, P<0.01). An inverse correlation between LDLR protein levels and plasma TC confirms the involvement of the LDLR in lowering of TC by alirocumab (R²=0.50, P<0.001).

### iii) Alirocumab does not Affect Liver Lipids and Fecal Bile Acid and Neutral Sterol Excretion

To evaluate the consequences of alirocumab-induced alterations in lipoprotein metabolism on hepatic lipid metabolism and excretion into feces, liver lipids and excretion of bile acids and neutral sterols in stool were determined. Alirocumab did not affect liver weight nor the hepatic content of cholesterol and TG, whereas atorvastatin and the combination treatments led to significant reductions in liver weight (-15%, P=0.067; -17%, P<0.05; -20%, P<0.01, respectively) and hepatic cholesteryl esters (-48%, P<0.01; -41%, P<0.01 and -44%, P<0.05, respectively) as compared to the control group, without a change in hepatic triglycerides (Table 4). Fecal output of bile acids and neutral sterols was not changed by the treatments (Table 5). These data indicate that despite the greater influx of cholesterol from the plasma compartment, hepatic cholesterol homeostasis is maintained during alirocumab and statin treatment.

**Table 4: Effect of alirocumab, atorvastatin and their combination on liver lipids.**

| | Liver lipids (µg/mg protein) | | |
|---|---|---|---|
| | FC | CE | TG |
| Control | 11.6 ± 1.6 | 50.6 ± 14.0 | 119.2 ± 33.3 |
| 3 mg alirocumab | 11.2 ± 1.4† | 48.2 ± 8.2 †† | 117.7 ± 21.6 |
| 10 mg alirocumab | 11.4 ± 2.0 † | 53.9 ± 10.4 ††† | 142.1 ± 43.0 † |
| Atorvastatin | 9.5 ± 0.9 * | 26.2 ± 4.8 ** | 90.6 ± 28.5 |
| 3 mg alirocumab + atorvastatin | 10.4 ± 1.8 | 29.6 ± 5.8 ** | 103.5 ± 36.8 |
| 10 mg alirocumab + atorvastatin | 10.7 ± 1.2 | 28.3 ± 9.0 * | 109.8 ± 28.8 |

| | | | |
|---|---|---|---|
| FC: free cholesterol; CE: cholesterol esters; TG: triglycerides. **P*<0.05, ***P*<0.01 as compared to control; †*P*<0.05, ††*P*<0.01, †††*P*<0.001 as compared to atorvastatin. | | | |

**Table 5: Effect of alirocumab, atorvastatin and their combination on neutral sterol and bile acid excretion.**

| | Neutral sterol excretion (µmol/100 g mouse/day) | Bile acid excretion (µmol/100 g mouse/day) |
|---|---|---|
| Control | 25.8 ± 5.5 | 13.5 ± 3.3 |
| 3 mg alirocumab | 20.4 ± 6.2 † | 14.3 ± 2.7 † |
| 10 mg alirocumab | 21.6 ± 5.6 † | 12.4 ± 3.2 |
| Atorvastatin | 30.3 ± 6.5 | 10.7 ± 2.4 |
| 3 mg alirocumab + atorvastatin | 28.6 ± 6.0 | 11.4 ± 2.2 |
| 10 mg alirocumab + atorvastatin | 27.5 ± 4.4 | 12.7 ± 1.6 |

| | | |
|---|---|---|
| †*P*<0.05 as compared to atorvastatin. | | |

### iv) Alirocumab Dose-Dependently Reduces Atherosclerosis Development and Enhances the Atheroprotective Effects of Atorvastatin

Effects of alirocumab on atherosclerosis development, in the absence and presence of atorvastatin, were assessed in the aortic root and arch after 18 weeks of treatment.

Representative images of atherosclerotic lesions, as illustrated in Figure 3, show that alirocumab, atorvastatin, and their combination reduced lesion progression. To confirm a reduction in atherosclerosis development, the lesion area and the number of lesions per cross-section were evaluated (Figure 4A and Figure 4B) along with lesion severity (Figure 4C). For the control group, total lesion area was 278 ± 89 x 103 µm2 per cross-section, which consisted of 4.0 ± 0.7 lesions per cross section. Alirocumab dose-dependently decreased atherosclerotic lesion size (-71%, P<0.001; -88%, P<0.001) and dose-dependently enhanced the effects of atorvastatin (-89%, P<0.001; -98%, P<0.001) as compared to the control. In addition, alirocumab, with and without atorvastatin, also decreased the number of lesions (-17%, P<0.05; -30%, P<0.01 and -41%, P<0.001; -77%, P<0.001, respectively). Mice treated with alirocumab, alone and in combination with atorvastatin, had more lesion-free sections and fewer severe (type IV-V) lesions compared with the control. Atorvastatin alone decreased lesion size (-35%, P<0.05) and reduced severity to a lesser extent with no effect on the number of lesions or undiseased segments. When compared to atorvastatin monotreatment, the combinations further decreased lesion size (-82%, P<0.001; -97%, P<0.001) and number of lesions (-38%, P<0.001; -76%, P<0.001) and increased the amount of undiseased segments.

To evaluate the effect of alirocumab treatment on lesion development at another spot along the aorta prone to development of atherosclerosis, plaque surface in the aortic arch was measured (Figure 4D). At this site, lesion development is delayed as compared with the aortic root. In line with the effects on atherogenesis in the aortic origin, 10 mg/kg alirocumab alone (-67%, P<0.05) and both doses together with atorvastatin (-73%, P<0.01; -73%, P<0.01%) reduced the total plaque area.

The anti-atherogenic effect of alirocumab and atorvastatin were evaluated (Figure 6) and a strong strong correlation between plasma TC levels and atherosclerotic lesion area in the aortic root was observed (R²=0.84, P<0.001; Figure 5), indicating an important role of cholesterol in the development of atherosclerosis.

### v) Alirocumab Reduces Monocyte Recruitment and Improves Lesion Stability Index

As a functional marker of vessel wall inflammation, the number of monocytes adhering to the activated endothelium in the aortic root area was counted and calculated per cross section (Figure 6A). In the control group, 1.9 ± 1.4 adhering monocytes were present. A marked reduction of adhering monocytes in both alirocumab groups of more than -48% (P<0.05 and P<0.01) and in the combination groups of more than -75% (both P<0.001) was found.

After investigating lesion morphology, treatment effects on plaque composition were analyzed in the severe lesions (type IV-V), which are considered to be the most vulnerable lesions. The macrophage area (Figure 6B) together with the necrotic core, including cholesterol clefts (Figure 6C), as destabilization factors, as well as SMC in the fibrotic cap (Figure 6D) and collagen area (Figure 6E) as stabilization factors were expressed as percentage of lesion area. Lesions in the control group consisted of 10.3% macrophages, 4.8% necrotic core and cholesterol clefts, 3.1% SMC in the cap and 48.4% collagen. Lesion stability index (Figure 6F) for the control group was 3.5 ± 0.8. When administered alone and in combination with atorvastatin, the lower dose of alirocumab (3 mg/kg) decreased necrotic content (-41%, P<0.05; -34%, P=0.052) and increased SMC content (+61%, P<0.05; +164%, P<0.001), whereas the higher dose (10 mg/kg) decreased macrophages (-36%, P<0.01; -70%, P<0.001) and necrotic content (-37%, P<0.05; -79%, P<0.001) and increased SMC (+130%, P<0.001; +122%, P=0.051) and collagen (+12%, P=0.067; +22% P<0.001) content. Alirocumab, therefore, improved lesion stability by reducing the destabilization factors and increasing the stabilization factors, as shown by an increase in lesion stability index alone (+24%, NS; +113%, P<0.01) and together with atorvastatin (+116%, P<0.05; 556%, P<0.001).

### vi) Safety Aspects of Alirocumab

No effects on body weight (gain) and food intake were noted in any treatment group as compared to control (data not shown). Plasma aspartate transaminase, as a potential marker of hepatocellular injury, was increased (1.9-fold, P=0.004) in the highest alirocumab dose group after 16 weeks of treatment (Table 3). However, both doses of alirocumab in combination with atorvastatin did not reveal similar results. The alirocumab on top of atorvastatin treatments both led to a significant reduction in liver weight as compared to the control group after 18 weeks of treatment (with 17%, P=0.026 and 20%, P=0.004, respectively), whereas monotreatment did not have an effect.

### Summary

The present study was designed to investigate the effects of alirocumab per se on atherosclerosis development and in combination with atorvastatin. Taken together, these data demonstrate that alirocumab dose-dependently decreases plasma cholesterol, progression of atherosclerosis and plaque vulnerability, and enhances the beneficial effects of atorvastatin in APOE*3Leiden.CETP mice.

### Embodiments of the invention:

One embodiment of the invention encompasses a method of inhibiting atherosclerotic plaque formation in a subject, the method comprising administering to the subject a pharmaceutical composition comprising a PCSK9 inhibitor.

One embodiment of the invention encompasses a pharmaceutical composition comprising a PCSK9 inhibitor for use in the inhibition of atherosclerotic plaque formation in a subject.

One embodiment of the invention encompasses said method or pharmaceutical composition, wherein the subject is non-hyperlipidemic.

One embodiment of the invention encompasses said method or pharmaceutical composition, wherein the subject is apparently healthy.

One embodiment of the invention encompasses a method of treating or inhibiting progression of atherosclerosis in a subject, the method comprising:
selecting a subject who has suffered a stroke or myocardial infarction; and
administering to the subject a pharmaceutical composition comprising a PCSK9 inhibitor, thereby treating or inhibiting progression of atherosclerosis.

One embodiment of the invention encompasses said method, wherein the subject is nonhyperlipidemic.

One embodiment of the invention encompasses a method of treating or inhibiting progression of atherosclerosis in a nonhyperlipidemic subject, the method comprising:
selecting a subject who has, or is known to be at risk of developing, atherosclerosis,
wherein the subject is non-hyperlipidemic; and
administering to the subject a pharmaceutical composition comprising a PCSK9 inhibitor, thereby treating or inhibiting progression of atherosclerosis.

One embodiment of the invention encompasses said method, wherein the subject is apparently healthy.

One embodiment of the invention encompasses said method or pharmaceutical composition, wherein the subject is nonhypercholesterolemic.

One embodiment of the invention encompasses said method or pharmaceutical composition, wherein the subject is nonhypertriglyceridemic.

One embodiment of the invention encompasses said method or pharmaceutical composition, wherein the subject has a disease or disorder selected from the group consisting of type I diabetes mellitus, type II diabetes mellitus, Kawasaki disease, chronic inflammatory disease, and hypertension.

One embodiment of the invention encompasses said method or pharmaceutical composition, wherein the subject has elevated levels of an inflammatory marker.

One embodiment of the invention encompasses said method or pharmaceutical composition, wherein the inflammatory marker is C-reactive protein.

One embodiment of the invention encompasses said method or pharmaceutical composition, wherein the inflammatory marker is an inflammatory cytokine.

One embodiment of the invention encompasses said method or pharmaceutical composition, wherein the PCSK9 inhibitor is an antibody or antigen binding protein that specifically binds to PCSK9.

One embodiment of the invention encompasses said method or pharmaceutical composition, wherein the antibody or antigen-binding protein comprises heavy and light chain CDR amino acid sequences having SEQ ID NOs:220, 222, 224, 228, 230 and 232.

One embodiment of the invention encompasses said method or pharmaceutical composition, wherein the antibody or antigen-binding protein comprises an HCVR having the amino acid sequence of SEQ ID NO:218 and an LCVR having the amino acid sequence of SEQ ID NO:226.

One embodiment of the invention encompasses said method or pharmaceutical composition, wherein the antibody or antigen-binding protein comprises heavy and light chain CDR amino acid sequences having SEQ ID NOs:76, 78, 80, 84, 86 and 88.

One embodiment of the invention encompasses said method or pharmaceutical composition, wherein the antibody or antigen-binding protein comprises an HCVR having the amino acid sequence of SEQ ID NO:90 and an LCVR having the amino acid sequence of SEQ ID NO:92.

One embodiment of the invention encompasses said method or pharmaceutical composition, wherein the antibody or antigen-binding protein binds to the same epitope on PCSK9 as an antibody comprising the heavy and light variable domain amino acid sequences forth in SEQ ID NOs: 90 and 92; or 218 and 216, respectively.

One embodiment of the invention encompasses said method or pharmaceutical composition, wherein the antibody or antigen-binding protein competes for binding to PCSK9 with an antibody comprising the heavy and light chain variable domain amino acid sequences forth in SEQ ID NOs: 90 and 92; or 218 and 216, respectively.

One embodiment of the invention encompasses said method or pharmaceutical composition , wherein the PCSK9 inhibitor reduces atherosclerotic plaque formation in the subject by at least 10%, 20%, 30%, 40%, 50%, 60%, 70%, 80%, or 90%.

One embodiment of the invention encompasses said method or pharmaceutical composition , wherein the subject has heterozygous Familial Hypercholesterolemia (heFH).

One embodiment of the invention encompasses said method or pharmaceutical composition, wherein the subject has a form of hypercholesterolemia that is not Familial Hypercholesterolemia (nonFH).

One embodiment of the invention encompasses said method or pharmaceutical composition, wherein the subject is on another lipid-modifyining agent before and/or during administration of the antibody or antigen-binding protein.

One embodiment of the invention encompasses said method or pharmaceutical composition, wherein the therapeutic lipid-modifying agent is selected from the group consisting of a statin, ezetimibe, a fibrate, niacin, an omega-3 fatty acid, and a bile acid resin.

One embodiment of the invention encompasses said method or pharmaceutical composition, wherein the statin is selected from the group consisting of cerivastatin, atorvastatin, simvastatin, pitavastatin, rosuvastatin, fluvastatin, lovastatin and pravastatin.

One embodiment of the invention encompasses said method or pharmaceuticalcomposition, wherein the subject is not on another lipid-modifying agent before and/or during administration of the antibody or antigen-binding protein.

One embodiment of the invention encompasses said method or pharmaceutical composition, wherein the antibody or antigen binding protein is administered subcutaneously.

One embodiment of the invention encompasses a unit dosage form comprising the pharmaceutical composition.

One embodiment of the invention encompasses said unit dosage form, wherein the PCSK9 inhibitor is an antibody or antigen-binding fragment and the unit dosage form comprises 75 mg, 150 mg, 200 mg, or 300 mg of the antibody or antigen-binding fragment.

One embodiment of the invention encompasses said unit dosage form, wherein the unit dosage form is selected from the group consisting of a pre-filled syringe, a pre-filled autoinjector, a vial, a cartridge, a reusable syringe, and a reusable autoinjector.

One embodiment of the invention encompasses said unit dosage form, wherein the unit dosage form is hermetically sealed.

One embodiment of the invention encompasses said unit dosage form, wherein the quantity of the antibody or antigen binding fragment is indicated on the hermetically-sealed dosage form.

One embodiment of the invention encompasses an article of manufacture comprising the pharmaceutical composition and a container.

One embodiment of the invention encompasses said article of manufacture comprising one or more unit dosage forms, and instructions for use.

One embodiment of the invention encompasses a use of the pharmaceutical composition in the manufacture of a medicament for treatment.

## Claims

1. A PCSK9 inhibitor for use in inhibiting atherosclerotic plaque formation in a subject.

2. A pharmaceutical composition comprising a PCSK9 inhibitor for use in the inhibition of atherosclerotic plaque formation in a subject.

3. The PCSK9 inhibitor of claim 1 or the pharmaceutical composition of claim 2, wherein the subject is non-hyperlipidemic.

4. The PCSK9 inhibitor of claim 1 or the pharmaceutical composition of claim 2, wherein the subject is apparently healthy.

5. A PCSK9 inhibitor or pharmaceutical composition comprising a PCSK9 inhibitor for use of treating or inhibiting progression of atherosclerosis in a subject, the use comprising the steps:
selecting a subject who has suffered a stroke or myocardial infarction; and
administering to the subject said PCSK9 inhibitor or a pharmaceutical composition comprising said PCSK9 inhibitor, thereby treating or inhibiting progression of atherosclerosis.

6. The PCSK9 inhibitor or pharmaceutical composition comprising a PCSK9 inhibitor of claim 5, wherein the subject is non-hyperlipidemic.

7. A PCSK9 inhibitor or pharmaceutical composition comprising a PCSK9 inhibitor for use of treating or inhibiting progression of atherosclerosis in a non-hyperlipidemic subject, the method comprising:
selecting a subject who has, or is known to be at risk of developing, atherosclerosis, wherein the subject is nonhyperlipidemic; and
administering to the subject said PCSK9 inhibitor or a pharmaceutical composition comprising said PCSK9 inhibitor, thereby treating or inhibiting progression of atherosclerosis.

8. The PCSK9 inhibitor or pharmaceutical composition comprising a PCSK9 inhibitor of claim 7, wherein the subject is apparently healthy.

9. The PCSK9 inhibitor or pharmaceutical composition comprising a PCSK9 inhibitor of any one of the preceding claims, wherein the subject is non-hypercholesterolemic.

10. The PCSK9 inhibitor or pharmaceutical composition comprising a PCSK9 inhibitor of any one of the preceding claims, wherein the subject is non-hypertriglyceridemic.

11. The PCSK9 inhibitor or pharmaceutical composition comprising a PCSK9 inhibitor of any one of the preceding claims, wherein the subject has a disease or disorder selected from the group consisting of type I diabetes mellitus, type II diabetes mellitus, Kawasaki disease, chronic inflammatory disease, and hypertension.

12. The PCSK9 inhibitor or pharmaceutical composition comprising a PCSK9 inhibitor of any one of the preceding claims, wherein the subject has elevated levels of an inflammatory marker.

13. The PCSK9 inhibitor or pharmaceutical composition comprising a PCSK9 inhibitor of claim 12, wherein the inflammatory marker is C-reactive protein.

14. The PCSK9 inhibitor or pharmaceutical composition comprising a PCSK9 inhibitor of claim 12, wherein the inflammatory marker is an inflammatory cytokine.

15. The PCSK9 inhibitor or pharmaceutical composition comprising a PCSK9 inhibitor of any one of the preceding claims, wherein the PCSK9 inhibitor is an antibody or antigen binding protein that specifically binds to PCSK9.

16. The PCSK9 inhibitor or pharmaceutical composition comprising a PCSK9 inhibitor of claim 15, wherein the antibody or antigen-binding protein comprises heavy and light chain CDR amino acid sequences having SEQ ID NOs: 220, 222, 224, 228, 230 and 232.

17. The PCSK9 inhibitor or pharmaceutical composition comprising a PCSK9 inhibitor of claim 16, wherein the antibody or antigen-binding protein comprises an HCVR having the amino acid sequence of SEQ ID NO:218 and an LCVR having the amino acid sequence of SEQ ID NO:226.

18. The PCSK9 inhibitor or pharmaceutical composition comprising a PCSK9 inhibitor of claim 15, wherein the antibody or antigen-binding protein comprises heavy and light chain CDR amino acid sequences having SEQ ID NOs:76, 78, 80, 84, 86 and 88.

19. The PCSK9 inhibitor or pharmaceutical composition comprising a PCSK9 inhibitor of claim 18, wherein the antibody or antigen-binding protein comprises an HCVR having the amino acid sequence of SEQ ID NO:90 and an LCVR having the amino acid sequence of SEQ ID NO:92.

20. The PCSK9 inhibitor or pharmaceutical composition comprising a PCSK9 inhibitor of any one of claims 15-19, wherein the antibody or antigen-binding protein binds to the same epitope on PCSK9 as an antibody comprising the heavy and light variable domain amino acid sequences forth in SEQ ID NOs: 90 and 92; or 218 and 216, respectively.

21. The PCSK9 inhibitor or pharmaceutical composition comprising a PCSK9 inhibitor of any one of claims 15-20, wherein the antibody or antigen-binding protein competes for binding to PCSK9 with an antibody comprising the heavy and light chain variable domain amino acid sequences forth in SEQ ID NOs: 90 and 92; or 218 and 216, respectively.

22. The PCSK9 inhibitor or pharmaceutical composition comprising a PCSK9 inhibitor of any one of the preceding claims, wherein the PCSK9 inhibitor reduces atherosclerotic plaque formation in the subject by at least 10%, 20%, 30%, 40%, 50%, 60%, 70%, 80%, or 90%.

23. The PCSK9 inhibitor or pharmaceutical composition comprising a PCSK9 inhibitor of any one of the preceding claims, wherein the subject has heterozygous Familial Hypercholesterolemia (heFH).

24. The PCSK9 inhibitor or pharmaceutical composition comprising a PCSK9 inhibitor of any one of the preceding claims, wherein the subject has a form of hypercholesterolemia that is not Familial Hypercholesterolemia (nonFH).

25. The PCSK9 inhibitor or pharmaceutical composition comprising a PCSK9 inhibitor of any one of the preceding claims, wherein the subject is on another lipid-modifyining agent before and/or during administration of the antibody or antigen-binding protein.

26. The PCSK9 inhibitor or pharmaceutical composition comprising a PCSK9 inhibitor of claim 25, wherein the therapeutic lipid-modifying agent is selected from the group consisting of a statin, ezetimibe, a fibrate, niacin, an omega-3 fatty acid, and a bile acid resin.

27. The PCSK9 inhibitor or pharmaceutical composition comprising a PCSK9 inhibitor of claim 26, wherein the statin is selected from the group consisting of cerivastatin, atorvastatin, simvastatin, pitavastatin, rosuvastatin, fluvastatin, lovastatin and pravastatin.

28. The PCSK9 inhibitor or pharmaceutical composition comprising a PCSK9 inhibitor of any one of the preceding claims, wherein the subject is not on another lipid-modifying agent before and/or during administration of the antibody or antigen-binding protein.

29. The PCSK9 inhibitor or pharmaceutical composition comprising a PCSK9 inhibitor of any one of the preceding claims, wherein the antibody or antigen binding protein is administered subcutaneously.

30. A unit dosage form comprising the PCSK9 inhibitor or the pharmaceutical composition of claim 15.

31. The unit dosage form of claim 30, wherein the PCSK9 inhibitor is an antibody or antigen-binding fragment and the unit dosage form comprises 75 mg, 150 mg, 200 mg, or 300 mg of the antibody or antigen-binding fragment.

32. The unit dosage form of claim 31, wherein the unit dosage form is selected from the group consisting of a pre-filled syringe, a pre-filled autoinjector, a vial, a cartridge, a reusable syringe, and a reusable autoinjector.

33. The unit dosage form of claim 32, wherein the unit dosage form is hermetically sealed.

34. The unit dosage form of any of claims 32-33, wherein the quantity of the antibody or antigen binding fragment is indicated on the hermetically-sealed dosage form.

35. An article of manufacture comprising the PCSK9 inhibitor or the pharmaceutical composition of claim 15 and a container.

36. The article of manufacture of claim 35 comprising one or more unit dosage forms according to claims 30-34, and instructions for use.

37. Use of the PCSK9 inhibitor or the pharmaceutical composition of any one of the preceding claims in the manufacture of a medicament for treatment.
